# EUROPEAN PATENT SPECIFICATION

(11) **EP 4 265 321 B1**
(45) Date of publication and mention of the grant of the patent: **18.03.2026**
(21) Application number: 22168738.7
(22) Date of filing: 19.04.2022
(51) Int. Cl.: B01D 53/30, G01N 33/00, G05B 23/02, B01D 46/79, G01N 1/22

(54) **SYSTEM FOR CONTINUOUS CONTROL OF A STATE OF A SAMPLE HANDLING DEVICE**
SYSTEM ZUM KONTINUIERLICHEN KONTROLLIEREN EINES ZUSTANDES EINER PROBENHANDHABUNGSVORRICHTUNG
SYSTÈME DE CONTRÔLE CONTINU DE L'ÉTAT D'UN DISPOSITIF DE MANIPULATION D'ÉCHANTILLON

(43) Date of publication of application: 25.10.2023
(73) Proprietor: ABB SCHWEIZ AG, 5400 Baden (CH)
(72) Inventor: BLEIL, Stefan, 61348 Bad Homburg (DE); BECKER, Christoph, 60599 Frankfurt am Main (DE); GIBBONS, Stephen, 65795 Hattersheim (DE); SYDES, Daniel, 53919 Weilerswist (DE)
(74) Representative: Maiwald GmbH

(56) References cited:
- WO-A1-2014/046286
- WO-A1-2020/053807
- JP-A- 2002 131 198

## Description

### Field of the invention

The present disclosure relates a system for continuous control of a state of a sampling handling device.

### Technical background

In continuous gas measurement systems, there is no suitable way to monitor a change in the components of the sample handling. A regular test gas application at the probe of a sample handling device is not accepted in the market and by the customers, because the maintenance and service effort is very high.

Thus, there is a need for improvement in continuously controlling a state of sample handling.

WO 2020/053807 A1 describes a method for monitoring a condition of a sample handling system of a gas analyser.

JP 2002 131198 A describes a method for waste gas measurement for analysing hazardous ingredients in the waste gas.

### Summary

The present invention is defined by the independent claims. Additional features of the invention are presented in the dependent claims.

According to an aspect of the invention a system for continuous control of a state of a sampling handling device comprises the following: An analyser device, configured for analysing a continuous gas sample. A sample handling device, configured for receiving a continuous gas sample and feeding the continuous gas sample to the analyser device. A liquid supplying device, configured for selectively supplying the sample handling device with a liquid. The liquid dissolves at least one solid component deposited on a filter of the sample handling device, thus creating a liquid additive. The sample handling device is configured for mixing the liquid additive with the continuous gas sample, thus providing a mixed continuous gas sample; wherein the sample handling device is configured for feeding the mixed continuous gas sample to the analyser device through a heated line. The analyser device is configured for analysing the fed mixed continuous gas sample, wherein analysing the mixed continuous gas sample comprises determining a gas composition of the fed mixed continuous gas sample. The analyser device is configured for determining a state of the sample handling device depending on the determined gas composition of the fed continuous gas sample.

Preferably, the term "state of a sample handling device", as used herein, relates to a condition of the sample handling device, in particular in view of a function or working performance of the sample handling device.

Preferably, the term "sample handling device", as used herein, relates to a device that is configured for receiving a continuous gas sample. The sample handling device is further configured for preparing a gas sample for an analyser device. The sample handling device preferably comprises a probe that is configured for receiving the continuous gas sample from a production process unit. The analyser device usually needs the gas sample in a specific condition and purity. The sample handling device for example comprises a filter that is configured for filtering the provided gas sample in order to filter out interfering substances like for example dust that might cause blockage of the sample handling device, in particular the probe, or the analyser device.

Preferably, the term "analyser device", as used herein, relates to a device that is configured for analysing a provided gas sample, in particular in view of a composition of the provided gas sample. In other words, the analyser device is configured for determining a composition of the gas sample in percent of each component of the gas sample and/or an amount of each component of the gas sample or a specific predetermined component of the gas sample.

Preferably, the heated line is configured for heating the continuous gas sample, or when present the mixed continuous gas sample, to a temperature that is specified by the analyser device for analysis of the provided gas sample.

The continuous gas sample is also referred to as process gas, because the continuous gas sample is a sample of a gas that is generated by a production process.

Preferably, the solid component that is deposited on the filter of the sample handling device is part of a gas composition of the continuous gas sample, even when no liquid is fed to the sample handling device. Furthermore, the solid component might also be deposited on a probe of the sample handling device.

Preferably, the sample handling device receives the continuous gas sample via a lance of a chimney, from which the continuous gas sample is ejected.

By adding the suitable liquid to the continuous gas sample, the gas composition can be influenced in such a way that this change can be determined by the analyser device. In other words, the analyser device is configured for determining a concentration and or a change in concentration of the solid component in the continuous gas sample that is used to determine the state of the sample handling device.

In other words, if the analyser device detects a change of the gas composition of the mixed continuous gas sample, in particular a significant change of any of the solid components, it is assumed that the sample handling device has been compromised by a deposition of solid components that is so high that it alters the state of the sample handling device. In other words, a correlation is considered between the amount of dissolved and washed out solid components by the liquid and the general amount of deposition of solid components on the sample handling device. For example, it is predetermined that a change of concentration of SO2 in the continuous gas sample of 50 ppm is only possible when the amount of deposited SO2 on the sample handling device is so high that the state of the sample handling device has been altered.

In a sampling system, solid components such as salts can form during continuous operation due to the gas composition of the continuous gas sample. These solid components can, for example, influence the function of the filter. By adding a liquid, these salts are dissolved and then produce a measurable signal in the analyzer. The strength, length and composition of this measurement signal provides information about the amount of salts and about the state of sample handling.

The described system allows to regularly check the state of the sample handling device while performing the regular analysis of the continuous gas sample.

Another advantage of the targeted addition of liquid is that the solid components, in particular salts, in the sample handling are washed out.

Thus, an improved system for continuous control of a state of sampling handling is provided.

In a preferred embodiment, analysing the mixed continuous gas sample comprises detecting a change in concentration of the components of the fed mixed continuous gas sample, wherein determining the change in concentration comprises comparing a concentration of the components of the continuous gas sample that is fed to the analyser when no liquid is added to the sample handling device with the concentration of the components of the mixed continuous gas sample.

In other words, the analyser device is configured for monitoring the concentration of the components of the continuous gas sample that is to be analysed. When the liquid is added to the continuous gas sample, the mixed continuous gas sample comprises the original continuous gas sample together with the vaporized liquid and the dissolved solid component. The analyser is preferably configured for monitoring a progression of the gas composition of the mixed continuous gas sample in view of the gas composition of the continuous gas sample.

In a preferred embodiment, a state of the sample handling device comprises an alert state; wherein the alert state is determined when the determined change in concentration of a component of the mixed continuous gas sample exceeds a predetermined alert threshold.

In a preferred embodiment, the predetermined alert threshold is defined at 100 ppm, preferably at 50 ppm.

Preferably, a concentration of the solid components in the mixed continuous gas sample is about 20 part per million, ppm. For example, due to a combustion process, the continuous gas sample always contains 20-30ppm SO2. Thus, in order to define by the analyser that the liquid has dissolved and washed out a significant amount of solid component that has altered the performance of the sample handling device, the concentration of the solid component has to change by a an amount of at least a plurality of 10 ppm. Thus, an alert is triggered by the analyser, when the concentration of one of the analysed solid components in the mixed continuous gas sample reaches the predefined threshold. The alert indicates to the user of the sample handling device that a state of the sample handling device has changed.

In a preferred embodiment, determining the gas composition comprises determining an amount of the at least one solid component dissolved by the liquid.

In a preferred embodiment, the analyser device is configured for determining a drift of sampling using the determined state of the sample handling device.

The analyser device allows determining in how far the sample handling device has been altered by the deposition of the solid components. Thus, when analysing the continuous gas sample, a drift can be predetermined based on the state of the sample handling device to counteract the difference in gas analysis that is caused by the deposition of the solid components.

Preferably, from changes over time of the gas composition, a drift for sampling can be calculated.

In a preferred embodiment, the analyser device is configured for determining a time difference between the supplying of the sample handling device with the liquid and of the detecting of the change in concentration of the components of the fed mixed continuous gas sample and determining a function of the sample handling device, in particular a probe of the sample handling device, using the determined time difference.

In a preferred embodiment, the sample handling device is supplied with the liquid for a predetermined amount of time.

The amount of time, the sample handling device is supplied with the liquid is preferably connected to an operation mode of the system. For example, in a measuring mode, the sample handling device is not supplied by the liquid and is only supplied by the continuous gas sample that is then provided to the analyser. A check mode can be activated, for example manually by a user or by a cyclic time window, in which the sample handling device is supplied with the liquid. The amount of time that the liquid is supplied to the sample handling device can also be a fixed predetermined value that is based on experience and is considered long enough to dissolve a significant portion of the deposited solid components on the sample handling device.

The sample handling device is supplied with the liquid controlled by the analyser device.

In a preferred embodiment, the liquid is a liquid that goes into a gas phase, when being heated by the heated line.

In a preferred embodiment, the liquid is water.

Water as the liquid allows dissolving most of the solid components on the filter, which preferably are salt compounds. Furthermore, water has a boiling point of 100°C. Thus, when mixed with the continuous gas sample and send to the analyser via the heated line, the water evaporates so that the analyser only receives gas components. In this case, the mixed continuous gas sample comprises of a process gas, which is continuous gas sample to be analysed in the first place, the water in gas phase and the dissolved solid components in gas phase.

In a preferred embodiment, the at least one solid component comprises a salt compound, preferably a NH4, SO2 and/or NO2 compound.

Preferably, the salt compound comprises ammonium salts that are salts that result from the reaction of ammonia and an acid within an aqueous solution. Instead of a metal atom (e.g. sodium), these salts contain a so-called ammonium group (NH4 +). These salts can be dissolved in water.

In a preferred embodiment, the heated line heats the mixed continuous gas sample to a temperature bigger than 100°C, preferably 180°C.

In a preferred embodiment, the filter of the sample handling device comprises silicon carbide.

Silicon carbide provides good filtering properties, however due to the roughness of the material, silicon carbide is prone for deposition of solid components on the filter.

According to another aspect of the invention, a method for continuous control of a state of sampling handling comprises the following steps: Supplying a sample handling device with a continuous gas sample. Selectively supplying the sample handling device with a liquid, which dissolves at least one solid component deposited on a filter of the sample handling device, thus creating a liquid additive. Mixing, by the sample handling device, the liquid additive with the continuous gas sample, thus providing a mixed continuous gas sample. Feeding, by the sample handling device, the mixed continuous gas sample to the analyser device through a heated line. Analysing the fed mixed continuous gas sample by the analyser device; wherein analysing the mixed continuous gas sample comprises determining a gas composition of the fed mixed continuous gas sample. Determining a state of the sample handling device depending on the determined gas composition of the fed continuous gas sample.

### Brief description of the drawings

The subject matter of the invention will be explained in more detail in the following text with reference to preferred exemplary embodiments which are illustrated in the attached drawings, in which:
- Fig. 1: schematically shows a system for continuous control of a state of a sample handling device;
- Fig. 2: schematically shows a method for continuous control of a state of a sample handling device.

Exemplary embodiments of the invention will be described in the following with reference to the accompanying drawings:

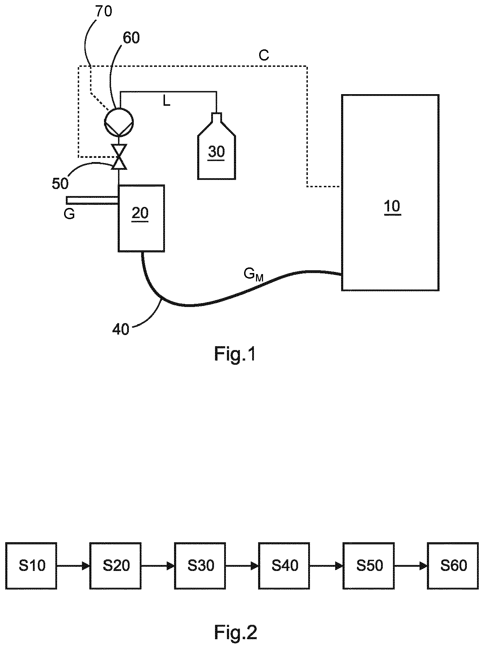

Fig. 1 describes a system for continuous control of a state of a sample handling device 20. The system comprises an analyser 10, a sample handling device 20, a liquid supplying device 30 and a heated line 40, connecting the sample handling device 20 with the analyser 10. Normally, the sample handling device 20 is provided with a continuous gas sample (G), which is provided by a lance (21), which is also called probe, of a chimney of a production process unit. The sample handling device 20 filters the continuous gas sample (G) and provides the continuous gas sample (G) via the heated line 40 to the analyser device. The continuous gas sample (G) is heated on its way to the analyser device, for example to up to 180°C. The analyser device 10 then analyses the continuous gas sample (G) in particular in view of the composition of the different components of the process gas. However, the sample handling device 20 itself needs to be regularly monitored as well, in particular in view of its status, or in other words of its performance.

The described analysis is controlled by the analyser device 10. In other words, the analyser device 10 is configured for managing different operating modes. In a measuring mode, only the continuous gas sample is provided to the analyser device 10. The system also comprises a liquid supplying device 30 that contains a liquid L, in particular water. The liquid supplying device 30 is connected to the sample handling device 20 through a pump 50 and a valve 60. When the pump 50 is activated, the pump 50 pumps the liquid L from the liquid supplying device 30 in direction of the sample handling device 20. When the pump 50 is activated, the valve 60 is opened to allow the liquid L to be pumped into the sample handling device 20.

The pump 50 and the valve 60 are controlled by the analyser device 10, in particular by a control signal C over a control line 70. In other words, the analyser device 10 can activate a check mode, different than the measuring mode, wherein in the check mode, the pump 50 is running and the valve 60 is opened, while in the measuring mode, the pump 50 is deactivated and the valve 60 is closed.

When the sample handling device 20 is supplied with the liquid L, the liquid L dissolves solid components that are deposited on the sample handling device 20, in particular a filter of the sample handling device 20. The sample handling device 20 thus mixes the provided continuous gas sample (G) with the provided liquid L and the dissolved solid components that are washed out of the sample handling device 20. Thus, the continuous gas sample (G) is enriched with additional components, namely the liquid L and the dissolved solid components. This enriched continuous gas sample (G) is referred to as mixed continuous gas sample (G_{M}). The mixed continuous gas sample (G_{M}) is then provided via the heated line 40 to the analyser unit 10. When heating the mixed continuous gas sample (G_{M}) in the heated line 40, the liquid L and the dissolved solid components go into a gas phase. Thus, the mixed continuous gas sample (G_{M}) only contains gas components when reaching the analyser device 10, which can be analysed by the analyser device 10.

If during check mode the liquid L in fact has dissolved solid components from the filter of the sample handling device 20, the analyser device 10 detects an increase in concentration of the solid component in the mixed continuous gas sample (G_{M}). For example, the solid component is SO2. SO2 is generally part of the continuous gas sample (G) due to combustion in the production process, but only has a concentration of about 20-30ppm. If SO2 was deposited on the filter of the sample handling device 20 and is dissolved by the liquid L, the analyser will for example detect a concentration of SO2 in the mixed continuous gas sample (G_{M}) of 100ppm. The analyser then concludes that the increased concentration of SO2 in the mixed continuous gas sample (G_{M}) is based on a certain amount of dissolved solid components on the filter of the sample handling device 20. Based on the time window of the check mode, in other words, the amount of time that the liquid L washes the filter of the sample handling device 20 and the measured progression of concentration of the dissolved solid components in the mixed continuous gas sample (G_{M}), the analyser evaluates how contaminated the filter of the sample handling device 20 might be and thus determined a state of the sample handling device 20.

Based on the determined state of the sample handling device 20, a user can be informed about the general state of sample handling or for example if a maintenance of the sample handling device 20 is necessary. Furthermore, the analyser device 10 can determine a drift that can be used to adjust the analysis of the continuous gas sample (G) in measuring mode.

If no change in the measured value is detected during the dosing of the liquid L, it can be assumed that the filter of the sample handling device, or for example a probe or the hot line 40 do not contain any soluble deposits.

Fig. 2 schematically shows a method for continuous control of a state of a sample handling device.

In a first step S10 a sample handling device 20 is supplied with a continuous gas sample (G). In a second step S20, the sample handling device 20 is selectively supplied with a liquid L, which dissolves at least one solid component deposited on a filter of the sample handling device 20, thus creating a liquid additive. In a third step S30, the sample handling device 20 mixes the liquid additive with the continuous gas sample (G), thus providing a mixed continuous gas sample (G_{M}). In a fourth step S40, the sample handling device 20 feeds the mixed continuous gas sample (G_{M}) to the analyser device through a heated line. In a fifth step S50, the fed mixed continuous gas sample (G_{M}) is sampled by the analyser device 10, wherein analysing the mixed continuous gas sample (G_{M}) comprises determining a gas composition of the fed mixed continuous gas sample (G_{M}). In a sixth step S60, a state of the sample handling device 20 is determined depending on the determined gas composition of the fed mixed continuous gas sample (G_{M}).

### List of reference symbols

- 10: analysing device
- 20: sample handling device
- 30: liquid supplying device
- 40: heated line
- 50: pump
- 60: valve
- 70: control line
- L: liquid
- G: continuous gas sample
- G_{M}: mixed continuous gas sample
- C: control signal
- S10: supplying a sample handling device with a continuous gas sample
- S20: selectively supplying the sample handling device with a liquid
- S30: mixing the liquid additive with the continuous gas sample
- S40: feeding the mixed continuous gas sample to the analyser device
- S50: analysing the fed mixed continuous gas
- S60: determining a state of the sample handling device

## Claims

1. A system for continuous control of a state of a sample handling device (20), comprising:
an analyser device (10), configured for analysing a continuous gas sample (G);
a sample handling device (20), configured for receiving the continuous gas sample (G) and feeding the continuous gas sample (G) to the analyser device (10);
a liquid supplying device (30), configured for selectively supplying the sample handling device (20) with a liquid (L);
wherein the liquid (L) dissolves at least one solid component deposited on a filter of the sample handling device (20), thus creating a liquid additive;
wherein the sample handling device (20) is configured for mixing the liquid additive with the continuous gas sample (G), thus providing a mixed continuous gas sample (G_{M}); wherein the sample handling device (20) is configured for feeding the mixed continuous gas sample (G_{M}) to the analyser device (10) through a heated line (40);
wherein the analyser device (10) is configured for analysing the fed mixed continuous gas sample (G_{M}), wherein analysing the mixed continuous gas sample (G_{M}) comprises determining a gas composition of the fed mixed continuous gas sample (G_{M});
wherein the analyser device is (10) configured for determining a state of the sample handling device (20) depending on the determined gas composition of the fed mixed continuous gas sample (G_{M});
wherein the sample handling device is supplied with the liquid (L) controlled by the analyser device.

2. System of claim 1,
wherein analysing the mixed continuous gas sample (G_{M}) comprises detecting a change in concentration of the components of the fed mixed continuous gas sample (G_{M}); wherein determining the change in concentration comprises comparing a concentration of the components of the continuous gas sample (G) that is fed to the analyser when no liquid (L) is added to the sample handling device with the concentration of the components of the mixed continuous gas sample (G_{M}).

3. System of claim 2,
wherein a state of the sample handling device comprises an alert state; wherein the alert state is determined when the determined change in concentration of a component of the mixed continuous gas sample (G_{M}) exceeds a predetermined alert threshold.

4. System of claim 3,
wherein the predetermined alert threshold is defined at 100 ppm, preferably at 50 ppm.

5. System of any of the preceding claims,
wherein determining the gas composition comprises determining an amount of the at least one solid component dissolved by the liquid (L).

6. System of any of the preceding claims,
wherein the analyser device (10) is configured for determining a drift of sampling using the determined state of the sample handling device (20).

7. System of any of the claims 2-4,
wherein the analyser device (10) is configured for determining a time difference between the supplying of the sample handling device with the liquid (L) and of the detecting of the change in concentration of the components of the fed mixed continuous gas sample (G_{M}) and determining a function of the sample handling device using the determined time difference.

8. System of any of the preceding claims,
wherein the sample handling device is supplied with the liquid (L) for a predetermined amount of time.

9. System of any of the preceding claims,
wherein the liquid (L) is a liquid (L) that goes into a gas phase, when being heated by the heated line.

10. System of any of the preceding claims,
wherein the liquid (L) is water.

11. System of any of the preceding claims,
wherein the at least one solid component comprises a salt compound, preferably a NH3, SO2 and/or NO2 compound.

12. System of any of the preceding claims,
wherein the heated line heats the mixed continuous gas sample (G_{M}) to a temperature bigger than 100°C, preferably 180°C.

13. System of any of the preceding claims,
wherein the filter of the sample handling device comprises silicon carbide.

14. Method for checking a state of sampling handling;
supplying (S10) a sample handling device (20) with a continuous gas sample (G);
selectively supplying (S20) the sample handling device (20) with a liquid (L), which dissolves at least one solid component deposited on a filter of the sample handling device (20), thus creating a liquid additive,
mixing (S30), by the sample handling device, the liquid additive with the continuous gas sample (G), thus providing a mixed continuous gas sample (G_{M});
feeding (S40), by the sample handling device (20), the mixed continuous gas sample (G_{M}) to the analyser device through a heated line;
analysing (S50), the fed mixed continuous gas sample (G_{M}) by the analyser device (10); wherein analysing the mixed continuous gas sample (G_{M}) comprises determining a gas composition of the fed mixed continuous gas sample (G_{M});
determining (S60), a state of the sample handling device (20) depending on the determined gas composition of the fed mixed continuous gas sample (G_{M}).

## Patentansprüche

1. System zur kontinuierlichen Steuerung des Zustands einer
Probenhandhabungsvorrichtung (20), umfassend:
eine Analysiervorrichtung (10), die dazu eingerichtet ist, eine kontinuierliche Gasprobe (G) zu analysieren;
eine Probenhandhabungsvorrichtung (20), die dazu eingerichtet ist, die kontinuierliche Gasprobe (G) aufzunehmen und diese zur Analysiervorrichtung (10) zu zuführen;
eine Flüssigkeitsversorgungseinrichtung (30), die dazu eingerichtet ist, die Probenhandhabungsvorrichtung (20) mit einer Flüssigkeit (L) zu versorgen;
wobei die Flüssigkeit (L) mindestens eine feste Komponente auflöst, die sich auf einem Filter der Probenhandhabungsvorrichtung (20) abgelagert hat, und somit ein flüssiges Additiv entsteht;
wobei die Probenhandhabungsvorrichtung (20) so eingerichtet ist, dass sie das flüssige Additiv mit der kontinuierlichen Gasprobe (G) mischt und somit eine gemischte kontinuierliche Gasprobe (G_{M}) bereitstellt; wobei die Probenhandhabungsvorrichtung (20) so eingerichtet ist, dass sie die gemischte kontinuierliche Gasprobe (G_{M}) über eine beheizte Leitung (40) der Analysiervorrichtung (10) zuführt;
wobei die Analysiervorrichtung (10) dazu eingerichtet ist, die gemischte kontinuierliche Gasprobe (G_{M}) zu analysieren, wobei das Analysieren der gemischten kontinuierlichen Gasprobe (G_{M}) das Bestimmen einer Gaszusammensetzung der zugeführten gemischten kontinuierlichen Gasprobe (G_{M}) umfasst;
wobei die Analysiervorrichtung (10) so eingerichtet ist, dass sie einen Zustand der Probenhandhabungsvorrichtung (20) abhängig von der bestimmten Gaszusammensetzung der zugeführten gemischten kontinuierlichen Gasprobe (G_{M}) bestimmt;
wobei die Probenhandhabungsvorrichtung mit der von der Analysiervorrichtung gesteuerten Flüssigkeit (L) versorgt wird.

2. System nach Anspruch 1,
wobei das Analysieren der gemischten kontinuierlichen Gasprobe (G_{M}) ein Detektieren einer Änderung der Konzentration der Komponenten der zugeführten gemischten kontinuierlichen Gasprobe (G_{M}) umfasst; wobei das Bestimmen der Konzentrationsänderung das Vergleichen einer Konzentration der Komponenten der kontinuierlichen Gasprobe (G), die dem Analysegerät zugeführt wird, wenn der Probenhandhabungsvorrichtung keine Flüssigkeit (L) zugeführt wird, mit der Konzentration der Komponenten der gemischten kontinuierlichen Gasprobe (G_{M}) umfasst.

3. System nach Anspruch 2,
wobei ein Zustand der Probenhandhabungsvorrichtung einen Alarmzustand umfasst; wobei der Alarmzustand bestimmt wird, wenn die bestimmte Konzentrationsänderung einer Komponente der gemischten kontinuierlichen Gasprobe (G_{M}) einen vorbestimmten Alarmschwellenwert überschreitet.

4. System nach Anspruch 3,
wobei der vorbestimmte Alarmschwellenwert auf 100 ppm, vorzugsweise auf 50 ppm, festgelegt ist.

5. System nach einem der vorangehenden Ansprüche,
wobei das Bestimmen der Gaszusammensetzung das Bestimmen einer Menge der mindestens einen festen Komponente umfasst, die durch die Flüssigkeit (L) gelöst ist.

6. System nach einem der vorangehenden Ansprüche,
wobei die Analysiervorrichtung (10) so eingerichtet ist, dass sie einen Drift des Sampelns unter Verwendung des bestimmten Zustands der Probenhandhabungsvorrichtung (20) bestimmt.

7. System nach einem der Ansprüche 2 bis 4,
wobei die Analysiervorrichtung (10) so eingerichtet ist, dass sie eine Zeitdifferenz zwischen der Versorgung der Probenhandhabungsvorrichtung mit der Flüssigkeit (L) und der Detektierung der Konzentrationsänderung der Komponenten der zugeführten gemischten kontinuierlichen Gasprobe (G_{M}) bestimmt und eine Funktion der Probenhandhabungsvorrichtung unter Verwendung der bestimmten Zeitdifferenz bestimmt.

8. System nach einem der vorangehenden Ansprüche,
wobei die Probenhandhabungsvorrichtung für eine vorbestimmte Zeitdauer mit der Flüssigkeit (L) versorgt wird.

9. System nach einem der vorangehenden Ansprüche,
wobei die Flüssigkeit (L) eine Flüssigkeit (L) ist, die in eine Gasphase übergeht, wenn sie durch die Heizleitung erwärmt wird.

10. System nach einem der vorangehenden Ansprüche,
wobei die Flüssigkeit (L) Wasser ist.

11. System nach einem der vorangehenden Ansprüche,
wobei die mindestens eine feste Komponente eine Salzverbindung, vorzugsweise eine NH3-, SO2- und/oder NO2-Verbindung, umfasst.

12. System nach einem der vorangehenden Ansprüche,
wobei die Heizleitung die gemischte kontinuierliche Gasprobe (G_{M}) auf eine Temperatur von mehr als 100 °C, vorzugsweise 180 °C, erhitzt.

13. System nach einem der vorangehenden Ansprüche,
wobei der Filter der Probenhandhabungsvorrichtung Siliziumkarbid umfasst.

14. Verfahren zum Überprüfen eines Zustands der Probenhandhabung;
Versorgen (S10) einer Probenhandhabungsvorrichtung (20) mit einer kontinuierlichen Gasprobe (G);
selektives Versorgen (S20) der Probenhandhabungsvorrichtung (20) mit einer Flüssigkeit (L), die mindestens eine auf einem Filter der Probenhandhabungsvorrichtung (20) abgelagerte feste Komponente löst, somit ein flüssiges Additiv entsteht,
Mischen (S30) des flüssigen Additivs mit der kontinuierlichen Gasprobe (G) durch die Probenhandhabungsvorrichtung, wodurch eine gemischte kontinuierliche Gasprobe (G_{M}) bereitgestellt wird;
Zuführen (S40) der gemischten kontinuierlichen Gasprobe (G_{M}) durch die Probenhandhabungsvorrichtung (20) zu der Analysiervorrichtung durch eine beheizte Leitung;
Analysieren (S50) der zugeführten gemischten kontinuierlichen Gasprobe (G_{M}) durch die Analysiervorrichtung (10); wobei das Analysieren der gemischten kontinuierlichen Gasprobe (G_{M}) das Bestimmen einer Gaszusammensetzung der zugeführten gemischten kontinuierlichen Gasprobe (G_{M}) umfasst;
Bestimmen (S60) eines Zustands der Probenhandhabungsvorrichtung (20) abhängig von der bestimmten Gaszusammensetzung der zugeführten gemischten kontinuierlichen Gasprobe (G_{M}).

## Revendications

1. Système destiné à contrôler en continu un état d'un dispositif de manipulation d'échantillon (20), comprenant :
un dispositif analyseur (10), conçu pour analyser un échantillon de gaz continu (G) ;
un dispositif de manipulation d'échantillon (20), conçu pour recevoir l'échantillon de gaz continu (G) et introduire l'échantillon de gaz continu (G) dans le dispositif analyseur (10) ;
un dispositif d'alimentation en liquide (30), conçu pour alimenter sélectivement le dispositif de manipulation d'échantillon (20) avec un liquide (L) ;
le liquide (L) dissolvant au moins un composant solide déposé sur un filtre du dispositif de manipulation d'échantillon (20), créant ainsi un additif liquide ;
le dispositif de manipulation d'échantillon (20) étant conçu pour mélanger l'additif liquide avec l'échantillon de gaz continu (G), permettant ainsi d'obtenir un échantillon de gaz continu mélangé (G_{M}) ; le dispositif de manipulation d'échantillon (20) étant conçu pour introduire l'échantillon de gaz continu mélangé (G_{M}) dans le dispositif analyseur (10) par une conduite chauffée (40) ;
le dispositif analyseur (10) étant conçu pour analyser l'échantillon de gaz continu mélangé introduit (G_{M}), l'analyse de l'échantillon de gaz continu mélangé (G_{M}) comprenant la détermination d'une composition gazeuse de l'échantillon de gaz continu mélangé introduit (G_{M}) ;
le dispositif analyseur (10) étant conçu pour déterminer un état du dispositif de manipulation d'échantillon (20) en fonction de la composition gazeuse déterminée de l'échantillon de gaz continu mélangé introduit (G_{M}) ;
le dispositif de manipulation d'échantillon étant alimenté avec le liquide (L) sous le contrôle du dispositif analyseur.

2. Système de la revendication 1,
dans lequel l'analyse de l'échantillon de gaz continu mélangé (G_{M}) comprend la détection d'une variation de concentration des composants de l'échantillon de gaz continu mélangé introduit (G_{M}) ; dans lequel la détermination de la variation de concentration comprend la comparaison d'une concentration des composants de l'échantillon de gaz continu (G) qui est introduit dans l'analyseur lorsqu'aucun liquide (L) n'est ajouté au dispositif de manipulation d'échantillon avec la concentration des composants de l'échantillon de gaz continu mélangé (G_{M}).

3. Système de la revendication 2,
dans lequel un état du dispositif de manipulation d'échantillon comprend un état d'alerte ; dans lequel l'état d'alerte est déterminé lorsque la variation déterminée de la concentration d'un composant de l'échantillon de gaz continu mélangé (G_{M}) dépasse un seuil d'alerte prédéterminé.

4. Système de la revendication 3,
dans lequel le seuil d'alerte prédéterminé est défini à 100 ppm, de préférence à 50 ppm.

5. Système de l'une quelconque des revendications précédentes,
dans lequel la détermination de la composition gazeuse comprend la détermination d'une quantité de l'au moins un composant solide dissous par le liquide (L).

6. Système de l'une quelconque des revendications précédentes,
dans lequel le dispositif analyseur (10) est conçu pour déterminer une dérive de l'échantillonnage en utilisant l'état déterminé du dispositif de manipulation d'échantillon (20).

7. Système de l'une quelconque des revendications 2 à 4,
dans lequel le dispositif analyseur (10) est conçu pour déterminer une différence de temps entre l'alimentation du dispositif de manipulation d'échantillon avec le liquide (L) et la détection de la variation de la concentration des composants de l'échantillon de gaz continu mélangé introduit (G_{M}) et déterminer une fonction du dispositif de manipulation d'échantillon en utilisant la différence de temps déterminée.

8. Système de l'une quelconque des revendications précédentes,
dans lequel le dispositif de manipulation d'échantillon est alimenté avec le liquide (L) pendant une durée prédéterminée.

9. Système de l'une quelconque des revendications précédentes,
dans lequel le liquide (L) est un liquide (L) qui passe en phase gazeuse lorsqu'il est chauffé par la conduite chauffée.

10. Système de l'une quelconque des revendications précédentes,
dans lequel le liquide (L) est l'eau.

11. Système de l'une quelconque des revendications précédentes,
dans lequel l'au moins un composant solide comprenant un composé salin, de préférence un composé de NH₃, SO₂ et/ou NO₂.

12. Système de l'une quelconque des revendications précédentes,
dans lequel la conduite chauffée chauffe l'échantillon de gaz continu mélangé (G_{M}) jusqu'à une température supérieure à 100 °C, de préférence 180 °C.

13. Système de l'une quelconque des revendications précédentes,
dans lequel le filtre du dispositif de manipulation d'échantillon comprend du carbure de silicium.

14. Procédé destiné à vérifier manipulation d'échantillon;
l'alimentation (S10) d'un dispositif de manipulation d'échantillon (20) avec un échantillon de gaz continu (G) ;
l'alimentation sélective (S20) du dispositif de manipulation d'échantillon (20) avec un liquide (L) qui dissout au moins un composant solide déposé sur un filtre du dispositif de manipulation d'échantillon (20), créant ainsi un additif liquide,
le mélange (S30), par le dispositif de manipulation d'échantillon, de l'additif liquide avec l'échantillon de gaz continu (G), ce qui permet ainsi d'obtenir un échantillon de gaz continu mélangé (G_{M}) ;
l'introduction (S40), par le dispositif de manipulation d'échantillon (20), de l'échantillon de gaz continu mélangé (G_{M}) dans le dispositif analyseur par une conduite chauffée ;
l'analyse (S50) de l'échantillon de gaz continu mélangé introduit (G_{M}) par le dispositif analyseur (10), l'analyse de l'échantillon de gaz continu mélangé (G_{M}) comprenant la détermination d'une composition gazeuse de l'échantillon de gaz continu mélangé introduit (G_{M}) ;
la détermination (S60) d'un état du dispositif de manipulation d'échantillon (20) en fonction de la composition gazeuse déterminée de l'échantillon de gaz continu mélangé introduit (G_{M}).
